# EUROPEAN PATENT APPLICATION

(11) **EP 4 776 301 A1**
(43) Date of publication of application: **15.07.2026**
(21) Application number: 25150710.9
(22) Date of filing: 08.01.2025
(51) Int. Cl.: G16H 40/67

(54) **SUBJECT MONITORING SYSTEM**

(71) Applicant: Koninklijke Philips N.V., 5656 AE Eindhoven (NL)
(72) Inventor: BULUT, Murtaza, 5656 AG Eindhoven (NL); GARCIA MORCHON, Oscar, 5656 AG Eindhoven (NL)
(74) Representative: Philips Intellectual Property & Standards

(57) **Abstract**

The present disclosure provides a subject monitoring system, and methods for processing an input to subject monitoring systems. A plurality of subject windows are displayed by the subject monitoring system, each presenting at least part of subject data associated with a respective subject. An input indicating a target action to be performed on subject data associated with a target subject window is received. Based on the input, an action is initiated to be performed on subject data associated with a further subject window amongst the subject windows. Accordingly, a need to repeat the input for the further subject windows is obviated by the automated application of actions across multiple subject windows, potentially saving time for clinicians and improving consistency in data handling across different subjects.

## Description

### FIELD OF THE INVENTION

The present invention relates to methods and systems for processing an input to a subject monitoring system, and more particularly for processing an input indicating a target action to be performed on subject data associated with a target subject window amongst a plurality of subject windows.

### BACKGROUND OF THE INVENTION

Central subject monitoring systems are essential in healthcare settings for continuous observation and management of subject data. These systems typically display real-time vital signs and other health metrics from multiple subjects, using a configuration of individual subject windows on a central monitor. Each subject window presents specific data related to a single subject, allowing healthcare providers to quickly assess and respond to subject needs. The systems are designed to integrate data from various medical devices, providing a comprehensive overview of subject statuses and facilitating immediate medical decision-making.

However, a particular focus is on improving ease and efficiency of use of such subject monitoring systems. Caregivers interact with these systems near-continuously to perform specific actions, such as adjusting alarm settings, updating display configurations, analysing subject data, adding annotations to subject data, monitoring protocol execution, and adapting workflows. With the large number of interactions required, providing inputs can become repetitive, which is time-consuming and prone to errors, especially in critical care environments where efficiency and accuracy are paramount.

It has been appreciated that an improved means for interacting with a subject monitoring system that improves efficiency and ease of use is desired.

### SUMMARY OF THE INVENTION

The invention is defined by the claims.

According to an aspect of the present disclosure, a method for processing an input to a subject monitoring system is provided. The method comprises: displaying a plurality of subject windows, each subject window presenting at least a part of subject data associated with a respective one of the plurality of subjects; receiving an input indicating a target action to be performed on subject data associated with a target subject window; and initiating an action to be performed on subject data associated with a further subject window amongst the subject windows based on the input.

This method allows for efficient and automated application of actions across multiple subject windows, potentially saving time for clinicians and improving consistency in data handling across different subjects. That is, the present invention provides a novel approach to managing and interacting with multiple subject windows in a subject monitoring system. By enabling actions performed on one subject window to be automatically applied or translated to other relevant subject windows, the invention significantly enhances the efficiency and consistency of data management in multi-subject monitoring scenarios.

A key aspect of disclosed embodiments is the concept of action translation across subject windows. When a user performs an action on a target subject window, the action may be analyzed to determine its applicability to other subject windows. This analysis can be performed at both low-level and high-level, allowing for precise matching of actions to compatible data characteristics as well as more flexible, intent-based application of actions across different subjects.

In other words, the present disclosure method provides for managing data in a subject monitoring system that displays multiple subject windows. Each subject window presents specific data related to a respective subject, such as a patient in a healthcare setting. Actions performed on one subject window responsive to an input may be automatically applied or translated to other relevant subject windows. This is achieved by initiating a similar or related action on subject data associated with a further subject window based on the received input. The disclosed method and system are designed to enhance the efficiency and consistency of data management across these subject windows.

It has been realised that a significant limitation in current subject monitoring systems is the isolated operation of actions within individual subject windows. When a specific action, such as adjusting alarm settings, adding annotations to data, or updating display configurations, is performed in one subject window, it does not automatically apply to other windows. This limitation requires repetitive manual input across multiple windows, which is time-consuming and prone to errors, especially in critical care environments where efficiency and accuracy are paramount. Accordingly, a key advantage provided by the disclosed method is the potential to significantly reduce repetitive manual inputs across multiple subject windows. This time-saving feature is particularly valuable in critical care environments where efficiency and accuracy are paramount, or in situations where demand exceeds capacity (e.g., when there is a staff shortage).

In some embodiments, the method may further comprise identifying the further subject window based on the received input.

Identifying the further subject window based on the input enables targeted application of actions to relevant subject windows, enhancing the efficiency of data management in multi-subject monitoring scenarios. By identifying the further subject window based on the received input, subject windows to which a same or similar input may be provided (i.e., to which a same or similar action may be applied) are identified. Accordingly, initiation of an action (e.g., a change in the visualization of the data, adding or removing data, etc.) to be performed on subject data to appropriate further subject windows may be achieved.

More specifically, identifying the further subject window may comprise generating a low-level action descriptor describing required characteristics of subject data for the target action to be performed; and identifying the further subject window based on the low-level action descriptor.

Using a low-level action descriptor allows for precise matching of actions to subject windows with compatible data characteristics, ensuring appropriate application of actions across different subjects. The low-level action descriptor may indicate a mapping between the input and the resulting change to the subject data. Accordingly, further subject windows to which a same of similar input and resulting change can be identified.

In some cases, identifying the further subject window based on the low-level action descriptor may comprise, for each of the plurality of subject windows: determining a suitability metric based on a comparison between characteristics of subject data associated with the respective subject window and the low-level action descriptor; and identifying the respective subject window as a further subject window responsive to the suitability metric meeting a predetermined condition.

This approach enables quantitative assessment of the suitability of each subject window for the target action, allowing for more refined selection of further subject windows. If characteristics of subject data of a subject window match well with the target action according to the low-level action descriptor as defined by the suitability metric, the subject window will be identified as a further subject window for initiation of a same or similar action (based on the input).

In some embodiments, the method may further comprise, for each subject window, modifying the suitability metric based on a comparison between characteristics of subject data associated with the respective subject window to a filtering descriptor describing predetermined criteria for characteristics of the subject window and/or characteristics of subject data associated with the subject window.

Modifying the suitability metric based on additional criteria allows for more nuanced selection of further subject windows, taking into account factors beyond just the compatibility with the target action. For instance, if the subject window is minimized (or otherwise not visible), then the subject window may not be identified as a further subject window. Of course, in other cases, the subject window may still be identified as a further subject window if the subject window is minimized. Similarly, if the subject data indicates that the subject is in a critical condition, then the associated subject window may not be identified as a further subject window.

In addition, the method may also comprise generating, with an input analysis algorithm, a high-level action descriptor describing an estimated intent associated with the input based on the low-level action descriptor; and identifying the further subject window based on the high-level action descriptor.

Using a high-level action descriptor allows for more flexible and intelligent identification of further subject windows based on the estimated intent of the user's action, potentially capturing a broader range of relevant subject windows. Thus, consideration is made not only are concrete required characteristics of subject data for the action to be performed, but the general intent of the change. For instance, if the action to be performed is a certain measurement, then the action may be described at a low-level as the particular measurement (e.g., a particular ECG measurement). However, at a high-level, the intent of the measurement may be described at a high level as the intended use of the measurement (e.g., ECG signal analysis). Then, identifying the further subject window can be based on the high-level intent (e.g., identify further subject windows associated with subject data to which ECG signal analysis may be performed).

This idea therefore leverages sophisticated algorithms to generate action descriptors and determine the suitability of actions for different subject windows. By considering various factors such as data characteristics, subject window properties, and user intent, the system can intelligently identify relevant subject windows and initiate appropriate actions. This approach allows for nuanced and context-aware application of actions, improving the relevance and usefulness of automated processes in subject monitoring.

In some embodiments, generating the high-level action descriptor may be further based on characteristics of subject data associated with the target subject window.

Incorporating characteristics of the target subject window's data into the high-level action descriptor generation process allows for more context-aware interpretation of the user's intent, and therefore a more accurate high-level action descriptor.

The action to be performed on subject data associated with the further subject window may be based on the high-level action descriptor.

Basing the action on the high-level action descriptor allows for more adaptive and context-appropriate actions to be applied to the further subject windows, potentially improving the relevance and usefulness of the automated actions. In other words, by basing the action to be initiated on the intent of the target action, a more appropriate action for the further subject window may be identified.

In some embodiments, the method of identifying the further subject window may be further based on characteristics of subject data associated with each of the plurality of subject windows, and/or characteristics of the subject window. Considering additional characteristics of subject data and subject windows in the identification process allows for more comprehensive and context-aware selection of further subject windows.

In exemplary embodiments, the method may further comprise associating each of the plurality of subject windows into one of a plurality of groups based on characteristics of subject data associated with the respective subject window and/or characteristics of the subject window; and identifying the further subject window based on the subject windows associated with the same group as the target subject window.

Grouping subject windows based on shared characteristics allows for more efficient identification of relevant further subject windows, potentially improving the speed and accuracy of action application across similar subjects. That is, the concept of subject window grouping based on shared characteristics enables more efficient identification of relevant windows for action application.

The method may further comprise initiating an action to be performed on subject data associated with the target subject window based on the input, and wherein initiating the action to be performed on subject data associated with the further subject window is performed simultaneously or sequentially to initiating the action to be performed on subject data associated with the target subject window.

This approach allows for flexible timing in the application of actions across multiple subject windows, enabling either immediate parallel processing or sequential processing as appropriate for the specific use case.

Alternatively, or in addition, the method may comprise initiating the action to be performed on subject data associated with the further subject window responsive to a predetermined trigger action. The predetermined trigger action may include receiving a further input describing an interaction with the further subject window.

Initiating actions based on predetermined triggers allows for more controlled and context-appropriate application of actions to further subject windows, potentially improving the relevance and timeliness of automated actions. More specifically, triggering actions based on interaction with the further subject window ensures that automated actions are applied at relevant and user-initiated moments, potentially improving the user experience and the contextual appropriateness of the actions.

According to a further aspect of the present disclosure, a computer program is provided. The computer program comprises computer program code means adapted, when said computer program is run on a computer, to implement a method for processing an input to a subject monitoring system as described herein.

According to another aspect of the present disclosure, a subject monitoring system is provided. The system comprises: a display configured to display a plurality of subject windows, each subject window presenting at least a part of subject data associated with a respective one of a plurality of subjects; and a controller configured to: receive an input indicating a target action to be performed on subject data associated with a target subject window; and initiate an action to be performed on subject data associated with a further subject window amongst the subject windows based on the input.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 illustrates a flowchart of a method for processing an input to a subject monitoring system, according to an embodiment;
FIG. 2 depicts a flowchart for identifying further subject windows in a monitoring system, according to aspects of the present disclosure;
FIG. 3 depicts a flowchart for identifying further subject windows in a monitoring system, according to further aspects of the present disclosure;
FIG. 4 shows a block diagram of a subject monitoring system comprising a controller and a display, according to an embodiment; and
FIG. 5 is a simplified block diagram of a computer within which one or more parts of an embodiment may be employed.

### DETAILED DESCRIPTION OF THE EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides a subject monitoring system, and methods for processing an input to subject monitoring systems. A plurality of subject windows are displayed by the subject monitoring system, each presenting at least part of subject data associated with a respective subject. An input indicating a target action to be performed on subject data associated with a target subject window is received. Based on the input, an action is initiated to be performed on subject data associated with a further subject window amongst the subject windows. Accordingly, a need to repeat the input for the further subject windows is obviated by the automated application of actions across multiple subject windows, potentially saving time for clinicians and improving consistency in data handling across different subjects.

The present disclosure provides a method and system for managing data in a subject monitoring system that displays multiple subject windows. Each subject window presents specific data related to a respective subject, such as a patient in a healthcare setting. The disclosed method and system are designed to enhance the efficiency and consistency of data management across these subject windows.

In particular, the disclosed method and system enable actions performed on one subject window to be automatically applied or translated to other relevant subject windows. This is achieved by receiving an input indicating a target action to be performed on subject data associated with a target subject window, and then initiating a similar or related action on subject data associated with a further subject window based on the received input.

The identification of the further subject window may be based on a low-level action descriptor describing required characteristics of subject data for the target action to be performed. In some cases, a high-level action descriptor describing an estimated intent associated with the input may also be generated and used to identify the further subject window.

This approach allows for precise matching of actions to compatible data characteristics as well as more flexible, intent-based application of actions across different subjects. It also enables quantitative assessment of the suitability of each subject window for the target action, allowing for more refined selection of further subject windows.

The disclosed method and system may provide several potential advantages. For instance, they may significantly reduce repetitive manual inputs across multiple subject windows, potentially saving time for clinicians and improving consistency in data handling across different subjects. They may also allow for more adaptive and context-appropriate actions to be applied to the further subject windows, potentially improving the relevance and usefulness of the automated actions.

By way of brief explanation, in centralized subject monitoring systems, information of multiple subjects is presented simultaneously or sequentially in the form of separate and independent subject sectors/window. Subject windows are bounded areas that show information specific to a single subject. The centralized system provides a quick overview of all monitored subjects, including vital signs and their waveforms, trends, as well as alarms, bed labels, subject names, screen notes, etc. Furthermore, the subject monitor may display various application windows that contain the clinical applications which are used to perform actions on subject data. The monitoring system may present the subject windows on a physical screen (e.g., a large monitor on a wall), or a virtual screen generated for virtual and augmented reality solutions.

The amount of information in a subject window depends on the size of the window. The layout can be changed by changing the configurations. Subject windows can be of different sizes and can be added or deleted as necessary. For example, windows for subjects with higher acuity can show more data (hence can be larger). In addition, subject windows may be displayed sequentially. That is, the screen of the subject monitor may cycle through various subject windows.

Multiple display options may be presented to the user which control the behavior of subject windows. For example, buttons may enable the minimizing of subject windows (which may create space to expand other subject windows, thus displaying more information), which may occur when active monitoring of the subject is paused. The subject window may be automatically re-established when monitoring of the subject resumes. Furthermore, the main screen may automatically resize subject windows based on availability of data. Specifically, with an automatic window resize option, if a window presents only one waveform, the window may resize to allow space for other subject windows.

Interaction with a subject window typically allows various actions to be performed on subject data associated with the subject of the subject window. Buttons in the window usually become visible when the cursor is moved inside the subject window, when the window is touched, or is otherwise interacted with. These buttons may enable the user to perform various actions, such as changing how subject data is displayed in the subject window, or to add or remove presented subject data from the subject window. Further, other actions may include the entry of clinical notes, adjustment of subject alarms, and the performance of analysis of subject data. In other words, the subject window facilitates interaction with applications for initiating the performance of various actions related to the subject and/or subject data. Subject data may be viewable via such applications.

In current solutions, actions performed in different subject windows are independent, and an action performed in one subject window does not influence the actions that are performed in other subject windows (i.e., actions are not automatically replicated or translated to the other subject windows). Thus, a user may provide an input to perform an action in one subject window but must provide the input to each subject window if a similar action is desired.

There are specific actions which may be desired to be applied to multiple subject windows saving valuable clinician time. Disclosed embodiments therefore describe a method to parametrically represent the performed action, to find other related subjects/subject windows, and to apply the action on multiple subject windows. Indeed, when viewed at high-level, there can be significant overlap between the data processing needs across different subjects, which means that prior actions may benefit current and future actions.

In proposed embodiments, actions performed in one subject window may be translated to other subject windows. In this way, when users start to work on data of another subject there will already be some analyses performed, enabling the user to continue the work, without having to start from the beginning. This can save work time and improve efficiency. More specifically, low-level action descriptors describing actions performed in one subject window may be processed to create high-level action descriptions. The high-level action descriptions may be used to identify other relevant subjects/subject windows to which a similar action may be applied and may be used to perform such actions automatically.

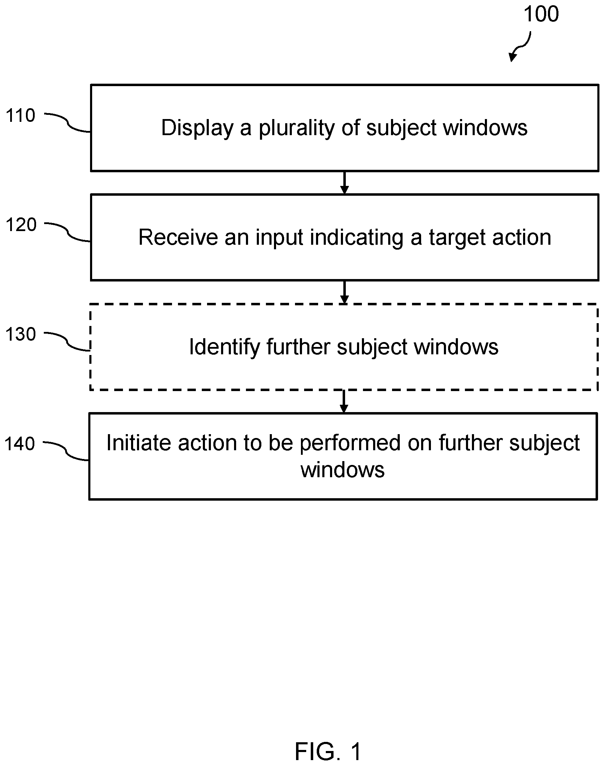

Referring to FIG. 1, a method 100 for processing an input for a subject monitoring system is presented. The method 100 enables actions to be performed on subject data of multiple subjects presented in respective subject windows of a subject monitoring system.

The method 100 begins with displaying 110 a plurality of subject windows. Each subject window presents at least a part of subject data associated with a respective one of the plurality of subjects. Accordingly, a user may monitor the plurality of subjects by observing the displayed subject windows, for example on a centralized display. This display may be on a computer monitor, a tablet screen, a smartphone screen, an augmented reality or virtual reality headset, or any other suitable display device. Each of the plurality of subject windows may be simultaneously displayed. In other cases, at least some of the subject windows may be sequentially displayed.

As described above, the subject data may include, but is not limited to, medical data (e.g. vital signs, known conditions), personal data (e.g., name, diagnostic group, assigned clinician), or any other relevant data associated with the subjects. Some of the subject data may be gathered in real-time from various sensors or other monitoring devices. The subject data may be obtained from electronic medical records. The subject data may be obtained from other subject monitoring devices and applications. Indeed, the subject monitoring system may be configured for two-way communication with monitoring devices and systems, database driven systems (such as electronic medical records, medication databases, etc.) and other applications (e.g., running on the cloud) that process and/or generate subject data. Of course, the subject monitoring system may be a central monitoring system that performs two-way communication with many devices and systems that the user may use to control the subject monitoring system.

The subject data may be presented in the form of waveforms, trends, notes, etc. Some of the subject data accessible to the subject monitoring system may not be presented. For example, some of the subject data associated with a subject related to a subject window may instead simply be stored ready to be referenced by a user. Alternatively, raw subject data may be used to derive information that is then displayed but is not immediately readable on the subject monitor.

The method 100 then involves receiving 120 an input indicating a target action to be performed on subject data associated with a target subject window. This input may be received from a user through various input devices such as a mouse, a keyboard, a touch screen, an application, or any other suitable input device. The input may involve a user clicking on a part of the display corresponding to the target subject window, hovering over the display etc. In some embodiments, the input may be received in the form of a voice input, a haptic input, a gesture, eye tracking, or another hands-free input. Alternatively, the input may be received in the form of a configuration file or based on a command from another subsystem.

The target action may include, but is not limited to, displaying specific subject data, modifying subject data, deleting subject data, displaying additional subject data, adding notes to the subject data, resizing or reformatting subject data, and/or performing analysis on subject data. By way of specific example, the target action may be to display a clinician name associated with the subject of the subject window, to modify alarm thresholds associated with the subject, to remove a particular waveform, to choose a different font size of a trend numeric, to add annotations to subject data in the subject window (e.g., caliper measurements), to make changes to workflows and/or protocols, etc. Essentially, the target action may be any action that can be performed by known subject monitoring systems.

To be clear, the target action may include a modification of the visualization of the subject data displayed in the subject window (e.g., changing the form of the visualization, or the data that is visualized), or a modification of the underlying subject data that is either displayed in the subject window, or associated with the subject of the subject window (e.g., adding, deleting or editing subject data, performing analysis on raw subject data).

In some aspects, the method may further include identifying 130 a further subject window based on the received input. This identification process may involve analyzing the received input and determining which subject windows are relevant for the target action. The identification process may be based on various factors such as the type of the target action, the characteristics of the subject data, the characteristics of the subject windows, or any other relevant factors.

Identifying 130 the further subject window may be based on characteristics of subject data and/or characteristics of the subject window. This means that the process can consider various factors when identifying the further subject window, such as the type of data presented in the subject window, the layout or configuration of the subject window, the status of the subject associated with the subject window, or any other relevant characteristics. This allows the process to select further subject windows that are most relevant or suitable for the target action, potentially improving the efficiency and effectiveness of the action replication.

In some embodiments, the method 100 may also include associating each of the plurality of subject windows into one of a plurality of groups based on characteristics of subject data associated with the respective subject window and/or characteristics of the subject window. This feature allows the system to categorize the subject windows into different groups, such as groups based on the type of data presented, the status of the subjects, the layout or configuration of the subject windows, or any other relevant factors. This grouping can facilitate the identification of relevant windows for action replication, as the system can focus on subject windows within the same group as the target subject window.

Once the subject windows are grouped, the process can identify 130 the further subject window based on the subject windows associated with the same group as the target subject window. This means that the system can select further subject windows from the same group as the target subject window for action replication. This approach can ensure that the actions are applied to subject windows with similar characteristics, potentially improving the consistency and relevance of the automated actions.

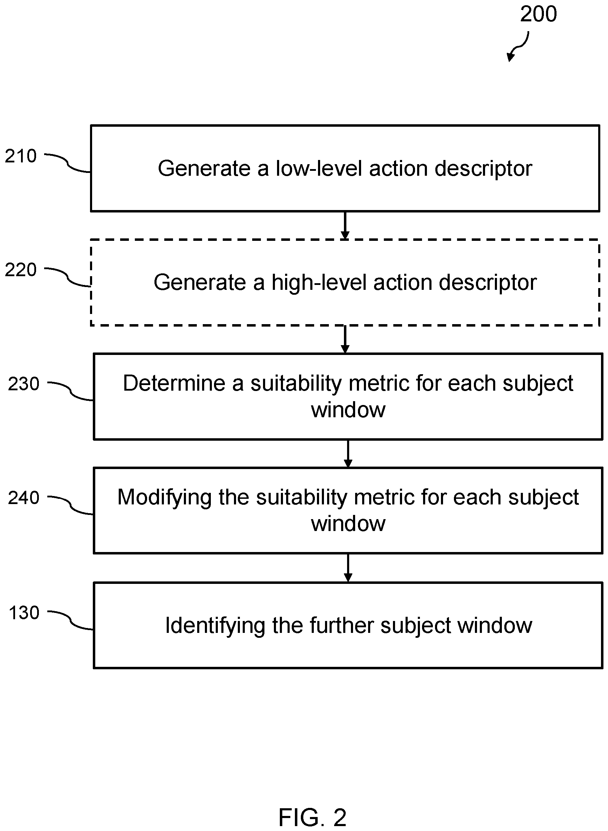

Further methods for identifying the subject window will be described in more detail below in reference to FIG. 2.

Nevertheless, in some cases identification of the further subject window may not be necessary. Indeed, all subject windows other than the target subject window may be considered to be further subject windows. Alternatively, the subject windows may be grouped, such that when an input is received for performing an action on subject data associated with a target subject window, all other subject windows in the same group as the target subject window may be considered to be further subject windows.

In any case, the method then involves initiating 140 an action to be performed on subject data associated with the further subject window based on the input. This action may be the same as the target action, or may be a similar action that is relevant to the subject data associated with the further subject window.

In some cases, the action is automatically performed on the subject data associated with the further subject window. In other cases, confirmation may be required to perform this further action. That is, initiating 140 the action may involve indicating the suggested action to be performed on the subject data associated with the further subject window, and triggering the action responsive to receiving a confirmation.

Furthermore, the action to be performed on subject data associated with the target subject window and the action to be performed on subject data associated with the further subject window may be performed simultaneously or sequentially. This allows for efficient and effective management of the subject data across multiple subject windows. That is, the target action on subject data of the target subject window, and the further action on subject data of the further subject window may be initiated at the same time, or subsequent to each other.

In some aspects, the action to be performed on subject data associated with the further subject window may be performed responsive to a predetermined trigger action.

This trigger action may include receiving a further input describing an interaction with the further subject window. The further input may be received through various input devices and may indicate various types of interactions such as selecting the further subject window, modifying the data in the further subject window, deleting the data in the further subject window, or any other interaction with the further subject window. In other words, the further action to be performed on the further subject window may only be initiated or triggered once the further subject window is interacted with.

Referring to FIG. 2, a process 200 for identifying a further subject window is presented.

The process begins with generating 210 a low-level action descriptor. This descriptor provides a detailed, parametric description of an action performed on a target subject window, particularly where there is a well-defined and concrete relation between the input and output. The low-level action descriptor describes the required characteristics of subject data for the target action to be performed. This may include, for example, the type of subject data, the format of the subject data, a size of the subject data visualized within the subject window, or any other relevant characteristics of the subject data including the way in which the subject data is visualized in the subject window.

In some aspects, the process may further include generating 220 a high-level action descriptor. This descriptor translates the low-level description into a broader, more generalized representation of the action's intent. The high-level action descriptor describes an estimated intent associated with the input based on the low-level action descriptor. This high-level action descriptor generation may also consider characteristics of subject data associated with the target subject window.

The generation of the high-level action descriptor may be achieved by use of a natural language generation (NLG) system that takes as input the raw data and metadata of the target action and/or associated low-level action descriptor (e.g., the time, location, type, parameters, and outcomes of the actions) and produces a natural language summary that describes the target action in a coherent and concise way. The NLG system can use various techniques, such as templates, rules, or neural networks, to generate the summary, and can also incorporate domain knowledge and user preferences to tailor the summary to the specific context and audience. The NLG system may also aggregate multiple target actions into a single summary, if they are related or sequential, and can provide different levels of granularity or detail depending on the user's needs. For example, a low level-action descriptor may indicate that the input has moved a start bar. By analysis of the action, and the low-level action descriptor, a high-level action descriptor may be generated that indicates that the input has adjusted the measurement interval to align with the peak of a displayed signal.

Of course, the high-level action descriptor may be generated using other systems suitable for translating the low-level action descriptor. For instance, large language models (such as ChatGPT) may be utilized to generate the high-level action descriptor.

The process then moves to determining 230 a suitability metric for each subject window. This step involves comparing the characteristics of subject data associated with each subject window with the requirements specified in the action descriptors. The suitability metric provides a quantitative measure of how well the subject data associated with each subject window matches the requirements for the target action. This allows the process to objectively assess the suitability of each subject window for the target action.

In some cases, the process may further include modifying 240 the suitability metric for each subject window. This modification step involves applying additional filtering criteria or adjusting the metric based on specific window or subject characteristics. The filtering descriptor describes predetermined criteria for characteristics of the subject window and/or characteristics of subject data associated with the subject window. This allows the process to refine the suitability metric based on additional context-specific factors, providing a more nuanced and accurate measure of suitability.

In yet further embodiments, modifying the suitability metric may be further based on context data describing a context of a user, subject, and/or system displaying the subject window. Thus, the modification may be context dependent (e.g., time-dependent). For instance, if a modification is required based on the context, the modification may be applied for 30 minutes. Once the context changes, the modification may no longer apply.

Finally, the process concludes with identifying 130 the further subject window. This step involves selecting one or more subject windows based on the modified suitability metrics. The further subject window is identified when the suitability metric meets a predetermined condition. This condition may be set based on various factors such as the type of the target action, the characteristics of the subject data, the characteristics of the subject windows, or any other relevant factors. This structured approach allows for precise and context-aware identification of further subject windows for action replication.

In some implementations of the above, the low-level action descriptor may be used to identify the further subject window(s), as well as to derive the action to be initiated in the further subject window. For instance, when a mouse is hovered over a caregiver icon by a user to show the name of the assigned caregiver in a target subject window, this may mean that the name of the assigned caregiver in the further subject windows is shown.

More specifically, the following steps may be performed to identify the further subject window(s) using a low-level action descriptor.
(i) The low-level action descriptor of the target action is used to determine the minimum requirements for performing the action. Typically, these requirements will be rules based and coded in the algorithm. For example, if the action is "displaying information when hovering over an icon", the requirement is that an icon is present in the further subject window;
(ii) Then, further subject windows are identified that satisfy the action requirements;
(iii) Additional filtering criteria may be applied, which can be based on characteristics of the subject window, subject data associated with the subject window, or workflow data. In some cases, there may be additional subject window specific, subject specific, or workflow specific requirements/restrictions which may be also considered. For instance, if the subject window is minimized, then the action may not be performed. For example, if the subject is in a different room the action may not be performed. For example, if clinician profiles and schedules do not overlap the action may not be performed.

For example, some actions that may be applied to further subject windows according to disclosed embodiments include the following:
(i) Input: Cursor hovering over a certain icon in a target subject window. Target action: Information associated with the icon (e.g., a caregiver icon, a bed icon, an equipment icon) in relation to the subject associated with the target subject window is shown. Action in further subject window(s): Information associated with the icon in relation to further subjects associated with the further subject windows are shown.
   In this case, the further subject windows may be identified as any subject windows associated with subject data that includes the information associated with the icon to be shown. There may be additional filtering criteria, such as requiring the further subject windows to not be minimized, for the further subject windows to be associated with subjects in the same class, room, etc. as the subject associated with the target subject window.
(ii) Input: Establishing a shortcut icon to appear in a target subject window. Target action: Generate a shortcut icon in the target subject window. Action in further subject window(s): Generate a shortcut icon in the further subject window(s).
(iii) Input: Change of an alarm limit in a target subject window. Target action: Change the alarm limit in the subject data associated with the target subject window. Action in further subject window(s): Suggest a change to alarm limits in the subject data associated with the further subject windows.
(iv) Input: Provide a set of display settings (e.g., window size, waveform size, waveform type, etc.) for a target subject window and subject data within the target subject window. Target action: apply the display settings to the target subject window. Action in further subject window(s): Suggest similar display settings for the further subject window(s) if the subject data to be displayed is similar.
(v) Input: Change selected subject data to be shown in the target subject window. Target action: Alter displayed subject data in target subject window. Action in further subject window(s): Suggest similar changes (or automatically change) the subject data in the further subject window.
(vi) Input: Entering annotation labels or other data in the target subject window. Target action: store the annotation labels and potentially display the annotation labels in target subject window. Action in further subject window(s): Suggest similar annotation labels in further subject windows to be stored as subject data of the subject associated with the further subject window(s). In further embodiments of the invention, and as described above, a high-level action descriptor may be generated from the low-level action descriptor. Using the same example as above, hovering the mouse over the caregiver icon implies that the user is trying to get some information about caregiver. In other words, it does not specifically indicate which information is shown, but from a high-level it is understood that some information about the caregiver is sought. Then, the high-level action descriptor (in this case "present information about the caregiver") is used to identify the further subject-window(s). In some cases, the high-level action descriptor is then used to generate specific low level action information for implementing the action in the further subject window.

More specifically, the following steps may be performed to identify the further subject window(s) using a high-level action descriptor:
(i) The low-level action descriptor is used to generate a high-level action description. Specifically, the low-level parametric representation of the action is used to generate a high-level descriptive representation of the action.
(ii) The high-level action descriptor is used to assess subject data of other subject windows, and to identify subject windows where the same high-level action can be applied.
(iii) Once identified, the high-level action descriptor can be used to generate a low-level action descriptor for the subject data associated with the identified further subject windows. That is, the specific subject data for the identified further subject window(s) is used to determine the specifications (rules) for the specific low-level further actions to be performed.

To summarize, a high-level action descriptor is generated that translates the low-level description into a broader, more generalized representation of the intent of the target action. The high-level descriptor is used to identify further subject windows associated with subject data to which the high-level action may be performed. The action to be performed on subject data associated with the further subject window is then based on the high-level action descriptor, potentially by translating the high-level action descriptor to a low-level action whilst considering the subject data of the further subject window. This means that the action initiated in the further subject window may not be an exact replication of the target action, but rather a related action that aligns with the broader intent of the target action.

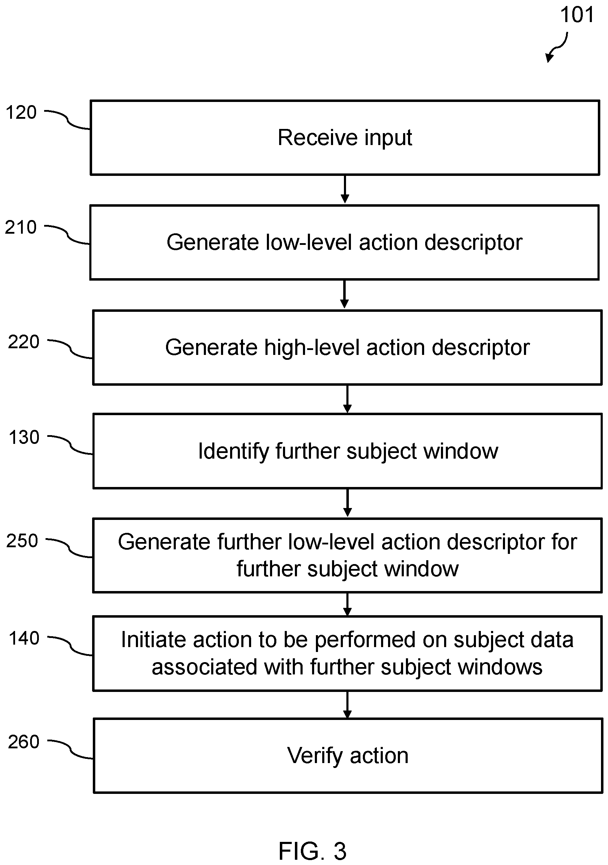

FIG. 3 depicts a method 101 for processing an input for a subject monitoring system that incorporates the above teaching.

In step 120, an input indicating a target action to be performed on subject data associated with a target subject window is received. This step is described in more detail above, and repeated description is omitted for sake of brevity.

In step 210, a low-level action descriptor is generated that provides a detailed, parametric description of an action performed on a target subject window. This defines the concrete relation between the input and an output. The low-level action descriptor describes the required characteristics of subject data for the target action to be performed. This may include, for example, the type of subject data, the format of the subject data, a size of the subject data visualized within the subject window, or any other relevant characteristics of the subject data including the way in which the subject data is visualized in the subject window.

In step 220, a high-level action descriptor is generated based on the low-level action descriptor. This descriptor translates the low-level description into a broader, more generalized representation of the action's intent. The high-level action descriptor may describe an estimated intent associated with the input based on the low-level action descriptor. That is, as described above, the low-level parametric representation of the action described by the input is used to generate a high-level descriptive representation of the action.

In step 130, one or more further subject windows are identified based on the high-level descriptor. The high-level action descriptor is used to assess subject data of other subject windows and subjects associated with said subject windows, and to identify further subject windows where the same high-level action can be applied.

In step 250, a further low-level action descriptor is generated for the identified further subject window(s). The further low-level action descriptor may describe the exact relationship between the input, and a resultant output for the particularly further subject window. This further low-level action descriptor may differ from the original low-level action descriptor due to differences between the target subject window and the further subject window. Accordingly, the specific patient data associated with the further subject window may be used to determine specifications (i.e., rules) for the specific low-level actions to be performed automatically on the further subject window. There may be one associated low-level action descriptor for each identified further subject window.

In step 140, an action to be performed on subject data associated with the further subject window is initiated. The action may be based on the generated further low-level action descriptors. Therefore, this action may differ somewhat from the intended action on the target subject window. Accordingly, the action may be appropriate for the specific individual subject data of the further subject window.

In step 260, the action to be performed on the further subject window may be verified. That is, in some cases, it may be advantageous to receive the approval of a user to execute the automatically generated action on the further subject window. The verification may be obtained by any known means.

By way of example, and to better illustrate an embodiment of the invention. The following example is provided that outlines a situation where ECG signal analysis is to be performed in a subject window.

Typically, a user may open a review application in a subject monitoring device to perform the ECG signal analysis. In this application, the user may create saved strip data, perform annotation actions, and perform caliper measurement actions on an ECG related to a particular subject. For saved strip data, the user may add labels and comments. Then, the user can perform caliper measurements for specific ECG signal parameters (such as PR, QRS, RR and QT intervals). The user can then store the strip data and the measurements.

These actions performed by the user may be considered an input in embodiment of the present invention. The action may be used to generate a low-level action descriptor. In the case of the present example, the low-level action descriptor may be "QT and QRS interval measurements". From this low-level action descriptor, a high-level action descriptor may be generated. For example, the high-level action descriptor may be "junction rhythm analysis" or "ECG signal analysis".

This high-level action descriptor may be used as the basis to identify one or more further subject windows. That is, this descriptor can be used to estimate other subjects for which the same actions (in terms of the high-level action, but not necessarily the same low-level action) can be performed. This identification may be achieved by:
(i) Estimating the reason for the action being performed on/in the initial subject window based on subject data, workflow data, and contextual information. For example, it may be that a specific ECG alarm was triggered, because of an upcoming team meeting, or because data needs to be entered into electronic medical records;
(ii) Identification of further subject windows associated with subject data of further subjects to which a similar (in terms of the high-level action descriptor) action may be performed, may then be based on the reason for the action (e.g., "save an ECG strip"), and the high-level action descriptor (e.g., "junction rhythm analysis"). For example, it may be determined that a similar ECG alarm is triggered for another subject, who is assigned the same clinician as the target subject. From these determinations, it may be anticipated that the user may also wish to perform a similar action on the further subjects; and
(iii) The user may also indicate other subjects, or other types of subjects (e.g., subjects with the same conditions, in the same room, etc.) for which a similar action should be performed.

Once the further subjects and/or subject windows are identified, the high-level action descriptor (e.g., create a saved ECG strip in electronic medical records for the subject) may be translated into a further low-level action descriptor that is specific to the further subject. For example, the low-level action descriptor may describe an action comprising the selection of a time window where the further subject experiences sinus arrhythmia, and the extraction of ECG signal parameters for the time window. Alternatively, the low-level action descriptor may describe an action comprising the presentation of ECG information in a given sequence.

Then, the action described by the low-level action descriptor may be initiated. This may comprise activation of a corresponding application for performing the action. For example, ECG analysis software may automatically find all time windows with sinus arrythmia and perform the appropriate measurements. The time boundary labels, as well as other generated outputs may then be stored.

Subsequently, when a user opens the further subject window associated with the further subject (or is prompted to do so) the user may be informed that pre-processed data is available. For instance, the regions with sinus arrythmia may be highlighted, when highlighted regions are selected the performed measurements and annotations may be presented. Further the user may review and alter the measurements.

By way of summary, this example shows how the invention may translate a low-level action descriptor performed on a target subject window (e.g., QT and QRS interval measurements) to a generalized high-level action descriptor (e.g., create ECG strip data to be saved in electronic medical records). Using the high-level action descriptor, further subjects may be identified, and specific low-level action descriptors may be generated for application to data associated with such subjects (e.g., detect and annotate periods with sinus arrhythmia). The further specific low-level action descripts can be used to initiate further actions on said subject data. The results may be presented for a user to review, update, and approve.

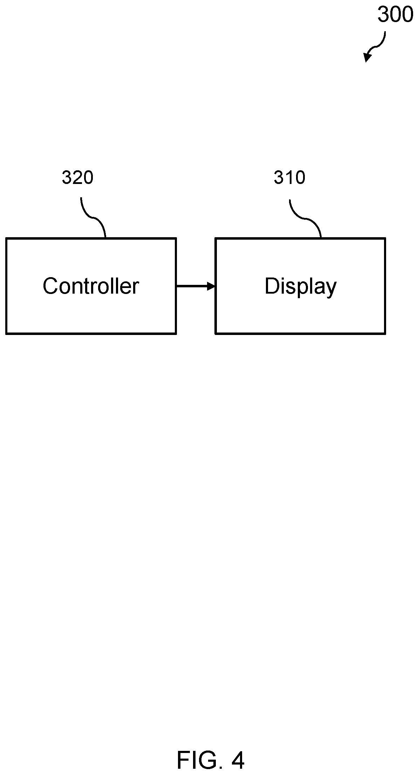

Moving onto FIG. 4, a subject monitoring system 300 is illustrated. The system 300 comprises two main components: a controller 320 and a display 310.

To be clear, the subject monitoring system may be any system, device or apparatus that has a controller and a display. To this end, the subject monitoring system may be a central subject monitoring system connected to a plurality of individual subject monitors (e.g., bedside monitors, ventilators, and other clinical equipment), sensors and actuators. Such a system may include a plurality of screens and/or computers. Nevertheless, the subject monitoring system as described herein may also simply be a single screen, tablet, computer, or other device comprising a controller 320 and a display 310. In this form, the subject monitoring system/device may be able to interact with multiple screens and/or subject monitors to change the subject windows displayed by said screens and/or subject monitors.

The controller 320 is communicatively coupled to the display 310, enabling the controller 320 to send information and commands to the display 310. The display 310 is configured to present visual information, which may include subject data in subject windows as described above. The subject data may include, but is not limited to, medical data, personal data, or any other relevant data associated with the subjects. The subject windows present at least a part of the subject data associated with a respective one of a plurality of subjects.

The controller 320 manages the overall operation of the system 300, processing inputs and determining actions to be performed on the subject data. In some aspects, the controller 320 is configured to receive an input indicating a target action to be performed on subject data associated with a target subject window.

Furthermore, the controller 320 is configured to initiate an action to be performed on subject data associated with a further subject window amongst the subject windows based on the input. This action may be the same as the target action or may be a different action that is relevant to the subject data associated with the further subject window.

In some aspects, the controller 320 may be configured to identify the further subject window based on the received input. As described above, this identification process may involve analyzing the received input and determining which subject windows are relevant for the target action. The identification process may be based on various factors such as the type of the target action, the characteristics of the subject data, the characteristics of the subject windows, or any other relevant factors.

In some aspects, the controller 320 of the subject monitoring system 300 may further include a clustering module. This module may be configured to group or cluster the subject windows (or indeed, the associated subjects) according to various criteria. These criteria may include, but are not limited to, the configuration of the subject window, the location of the subject (e.g., subject bed location), the profile of the clinician assigned to the subject, the health status of the subject, or any other relevant factors. This clustering feature allows the system 300 to organize the subject windows in a way that reflects the similarities or differences among the subjects, potentially improving the efficiency and effectiveness of the action replication.

For instance, the clustering module may group subject windows based on the state of the subject window. Subject windows displaying similar types of data or presenting data in a similar format may be grouped together. Similarly, subject windows associated with subjects in the same location, such as the same hospital ward or the same home care setting, may be grouped together. Subject windows associated with subjects being cared for by the same clinician or team of clinicians may also be grouped together. Furthermore, subject windows associated with subjects having similar health statuses, such as similar diagnoses or similar treatment plans, may be grouped together.

In some cases, the user of the system 300 may be able to determine which features are used to cluster the subject windows. The user may select the criteria for clustering through a user interface provided by the system 300. This user-defined clustering feature allows the user to customize the organization of the subject windows according to their specific needs or preferences, potentially improving the usability and adaptability of the system 300.

Once the subject windows are grouped or clustered, the controller 320 may identify the further subject window based on the subject windows associated with the same group as the target subject window. This means that the system 300 can select further subject windows from the same group as the target subject window for action replication. This approach can ensure that the actions are applied to subject windows with similar characteristics, potentially improving the consistency and relevance of the automated actions.

In some aspects, the subject monitoring system 300 may receive inputs through various modalities. For example, the system 300 may be configured to receive inputs through eye tracking, speech recognition, gesture recognition, or a smart phone interface. These alternative input modalities may provide additional flexibility and convenience for the user, potentially improving the usability and efficiency of the system 300. For instance, eye tracking may allow the user to interact with the subject windows by simply looking at specific areas or elements within the windows. Speech recognition may allow the user to perform actions or make selections by speaking commands. Gesture recognition may allow the user to interact with the subject windows by making specific hand or body movements. A smart phone interface may allow the user to interact with the subject windows remotely, providing additional convenience and flexibility.

Overall, the subject monitoring system 300 provides a flexible and efficient platform for processing an input to a subject monitoring system and managing data of multiple subjects presented in respective subject windows. The system 300 allows for actions performed on one subject window to be automatically applied or translated to other relevant subject windows, enhancing efficiency in managing multiple subjects simultaneously.

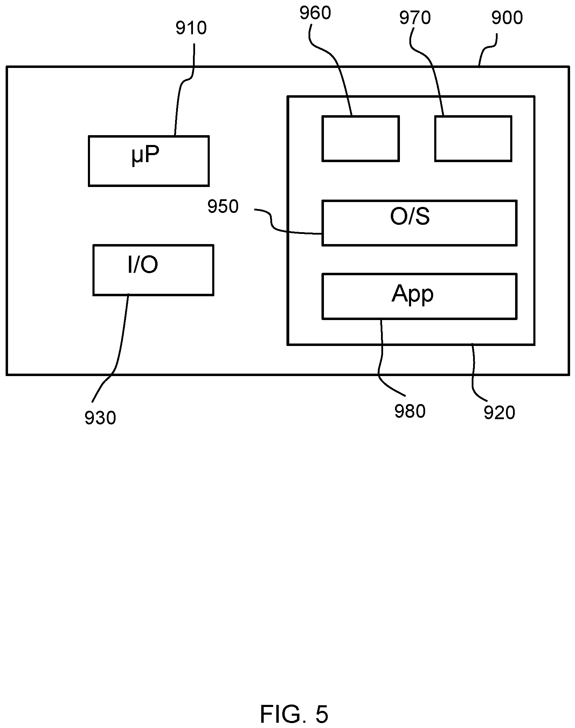

FIG. 5 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer-related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method for processing an input to a subject monitoring system, the method comprising:
displaying (110) a plurality of subject windows, each subject window presenting at least a part of subject data associated with a respective one of the plurality of subjects;
receiving (120) an input indicating a target action to be performed on subject data associated with a target subject window; and
initiating (140) an action to be performed on subject data associated with a further subject window amongst the subject windows based on the input.

2. The method of claim 1, further comprising:
identifying (130) the further subject window based on the received input.

3. The method of claim 1 or 2, wherein identifying the further subject window comprises:
generating (210) a low-level action descriptor describing required characteristics of subject data for the target action to be performed;
identifying (130) the further subject window based the low-level action descriptor.

4. The method of claim 3, wherein identifying the further subject window based on the low-level action descriptor comprises, for each of the plurality of subject windows:
determining (230) a suitability metric based on a comparison between characteristics of subject data associated with the respective subject window and the low-level action descriptor; and
identifying (130) the respective subject window as a further subject window responsive to the suitability metric meeting a predetermined condition.

5. The method of claim 4, further comprising, for each subject window, modifying (240) the suitability metric based on a comparison between characteristics of subject data associated with the respective subject window to a filtering descriptor describing predetermined criteria for characteristics of the subject window and/or characteristics of subject data associated with the subject window.

6. The method of any of claims 3-5, further comprising:
generating (220), with an input analysis algorithm, a high-level action descriptor describing an estimated intent associated with the input based on the low-level action descriptor; and
identifying (130) the further subject window based on the high-level action descriptor.

7. The method of claim 6, wherein generating (220) the high-level action descriptor is further based on characteristics of subject data associated with the target subject window.

8. The method of claim 6 or 7, wherein the action to be performed on subject data associated with the further subject window is based on the high-level action descriptor.

9. The method of any of claims 2-8, wherein identifying (130) the further subject window is further based on characteristics of subject data associated with each of the plurality of subject windows, and/or characteristics of the subject window.

10. The method of any of claims 2-9, further comprising associating each of the plurality of subject windows into one of a plurality of groups based on characteristics of subject data associated with the respective subject window and/or characteristics of the subject window; and
identifying (130) the further subject window based on the subject windows associated with the same group as the target subject window.

11. The method of any of claims 1-10, further comprising initiating an action to be performed on subject data associated with the target subject window based on the input, and wherein initiating (140) the action to be performed on subject data associated with the further subject window is performed simultaneously or sequentially to initiating the action to be performed on subject data associated with the target subject window.

12. The method of any of claims 1-10, wherein initiating (140) the action to be performed on subject data associated with the further subject window is performed responsive to a predetermined trigger action.

13. The method of claim 12, wherein the predetermined trigger action includes receiving a further input describing an interaction with the further subject window.

14. A computer program comprising computer program code means adapted, when said computer program is run on a computer, to implement the method of any of claims 1-13.

15. A subject monitoring system (300), comprising:
a display (310) configured to display a plurality of subject windows, each subject window presenting at least a part of subject data associated with a respective one of a plurality of subjects; and
a controller (320) configured to:
receive an input indicating a target action to be performed on subject data associated with a target subject window; and
initiate an action to be performed on subject data associated with a further subject window amongst the subject windows based on the input.
